Europäisches Patentamt
European Patent Office
Office européen des brevets

(11) Publication number: 0 293 529 A1

# EUROPEAN PATENT APPLICATION

(21) Application number: 87305528.9

(22) Date of filing: 22.06.87

(51) Int. Cl.4: A61K 31/615 , A61K 31/19 , //(A61K31/19,31:13)

(30) Priority: 02.06.87 GB 8712921

(43) Date of publication of application: 07.12.88 Bulletin 88/49

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Phillips, Anthony J.
Moredown Standon Road Little Hadham
Ware Hertfordshire SG11 2DD(GB)

(74) Representative: Hesketh, Alan, Dr.
European Patent Department Merck & Co., Inc. Terlings Park Eastwick Road
Harlow Essex, CM20 2QR(GB)

(54) **Admixtures of diflunisal and tromethamine.**

(57) An admixture of diflunisal, i.e.,

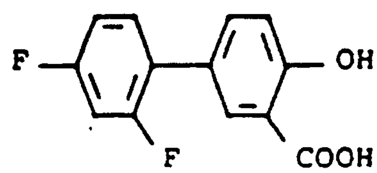

and tromethamine, i.e., $NH_2C(CH_2OH)_3$ has been found to exhibit improved absorption rate and therefore shortened onset of activity.

EP 0 293 529 A1

Xerox Copy Centre

# ADMIXTURES OF DIFLUNISAL AND TROMETHAMINE

## BACKGROUND OF THE INVENTION

Diflunisal, 2′,4′-difluoro-4-hydroxy[1,1′-biphenyl]-3-carboxylic acid, has been shown to exhibit analgesic and anti-inflammatory activity in humans. Diflunisal, however, has a slow onset of activity. It was surprisingly found that the compositions of the present invention shorten the onset of activity.

## SUMMARY OF THE INVENTION

This invention relates to admixtures of diflunisal and tromethamine. Diflunisal is disclosed in U.S. Patent 3,714,226, which is hereby incorporated by reference. The admixture of the present invention markedly increased solubility and dissolution rate under aqueous conditions and improved the pharmacokinetic profile when compared with diflunisal alone.

## DETAILED DESCRIPTION OF THE INVENTION

The present invention is directed to an admixture of diflunisal and tromethamine. Diflunisal may be represented by the following structural formula (A):

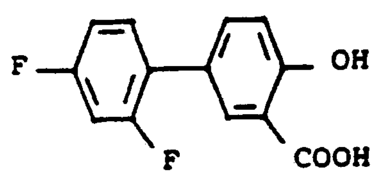

Tromethamine may be represented by the following structure (B): $NH_2C(CH_2OH)_3$.

The admixture may optionally be used in combination with pharmaceutical excipients.

This invention also relates to a method of treating inflammation in patients using the admixture.

The admixture of the instant invention can be used to treat inflammation by reducing inflammation and relieving pain in such diseases as rheumatoid arthritis, osteoarthritis, gout, infectious arthritis and rheumatic fever. Furthermore, the admixture of the instant invention has better potency at the same dosage levels than similar type compounds known in the prior art and exhibits a lower incidence of side effects.

The admixture also has analgesic activity and would be administered and used in the same manner and in the same dosage ranges as if they were being used to treat inflammation as discussed further on.

The admixture exhibits in addition to potent anti-inflammatory effects a smaller incidence of vomiting (emesis effect) than do similar type compounds of the prior art, especially acetyl salicylic acid (aspirin) type compounds. The admixture of the instant invention has a better therapeutic ratio than does aspirin.

The treatment of inflammation in accordance with the method of the present invention is accomplished by orally administering to patients a therapeutic dose of the admixture, particularly with a non-toxic pharmaceutically acceptable excipient, preferably in tablet or capsule form.

The non-toxic pharmaceutical excipient may be for example, either a solid or a liquid. Exemplary of solid excipient are lactose, corn starch, gelatin, talc, terotex, stearic acid, magnesium stearate, terra alba, sucrose, agar, pectin, cab-o-sil, and acacia. Exemplary of liquid excipients are peanut oil, olive oil, sesame oil and water.

Several pharmaceutical forms of the therapeutically useful admixture can be used. For example, if a solid excipient is used, the admixture may take the form of tablets, capsules, powders, troches or lozenges, prepared by standard pharmaceutical techniques. If a liquid excipient is used, the preparation may be in the form of a soft gelatin capsule, a syrup or a liquid suspension.

The admixture is used in an amount sufficient to treat inflammation, that is to reduce inflammation. Advantageously, the admixture will contain the active ingredient, namely, diflunisal in an amount of from about 1 mg to 35 mg per kg body weight per day (50 mg to 2.5 g per patient per day), preferably from about 2 mg to 14 mg per kg body weight per day (100 mg to 1 g per patient per day).

The method of treatment of this invention comprises internally administering to a patient (animal or human), the admixture, particularly admixed with a non-toxic pharmaceutical excipient such as exemplified above. The most rapid and effective anti-inflammatory effect is obtained from oral administration of a daily dosage of from about 4 to 10 mg/kg/day. It should be understood, however, that although preferred dosage ranges are given, the dose level for any particular patient depends upon the age, body weight, sex, time of administration, route of administration, rate of excretion, drug combination, reaction sensitivities and severity of the particular disease of the patient.

Diflunisal and tromethamine are known compounds and are prepared according to published procedures.

The admixture may conveniently comprise from 125 to 500 mg of diflunisal and 50 to 905 mg of tromethamine.

It is preferred that the admixture comprises about 200 to 500 mg of diflunisal and about 50 to 670 mg of tromethamine and that the weight ratio of diflunisal to tromethamine be about 10:1 to about 1:3.3. Preferably the admixture comprises 125 - 500 mg of diflusinal and 50 to 424 mg of tromethamine; especially 200 - 500 mg diflusinal and 100 to 424 mg of tromethamine.

The admixture may be prepared by blending diflunisal, tromethamine and a suitable binding agent in a suitable mixer and granulated by addition of water or aqueous-solvent mixtures. The wet mass may be milled, dried, rescreened and blended with disintegrants and lubricants before compression. To prevent the irritancy of diflunisal to the mouth and throat, the tablets may be film coated.

The following Example is used by way of illustration:

## EXAMPLE 1

This was an open-label, single-dose, two-way crossover study in 18 healthy subjects. Sixteen completed the following treatments. Treatment days were separated by at least 7 days.

Treatment A:

One diflunisal-tromethamine tablet containing 250 mg (1 mEq) diflunisal and 550 mg (4.5 mEq) tromethamine

Treatment B:

One DOLOBID (manufactured by Merck & Co. Inc.) tablet containing 250 mg diflunisal

Plasma samples were collected at 0, 0.25, 0.5, 0.75, 1, 1.5, 2, 3, 4, and 6 hours. Complete urine was collected for the following intervals: -1 to 0 hour (control), 0-2, 2-4, 4-6, 6-24, 24-48, 48-72, and 72-96 hours. All samples were kept frozen until the time of analysis.

Table 1 lists the physicochemical properties of the two dosage forms. Table 2 summarizes the pharmacokinetic parameters. Based on the date shown, it is apparent that the total uirnary recovery of diflunisal and the peak plasma level of diflunisal are comparable between the two treatments. However, the time-to-plasma peak appears to be shifted to the left, suggesting an improvement in the absorption rate. The extent of absorption, as judged by the total urinery recoveries, was not affected.

Table 1

Physicochemical Properties of the Test Dosage Forms

| | DOLOBID Tablets | Diflunisal-Tromethamine Tablets |
|---|---|---|
| Composition | | |
| Diflunisal | 250 mg | 250 mg |
| Hydroxypropylcellulose (binding agent) LF NF w/0.3% silica | 8.5 mg | -- |
| Starch Pregelatinized NF 1500 (bulking agent/disintegrant) | 104 mg | -- |
| Cellulose Microcrystalline NF (bulking agent/disintegrant) | 43.5 mg | -- |
| Magnesium Stearate NF (lubricant) | 4 mg | 5.25 mg |
| Coating | 10 mg | -- |
| Tromethamine | -- | 550 mg |
| Byco C Gelatin (binding agent) | -- | 40 mg |
| Purified Talc (anti-adherent) | -- | 35 mg |
| Explotab (disintegrant) | -- | 35 mg |
| Coating | | |
| Hydroxypropylmethylcellulose | -- | 5 mg |
| Polyethylene Glycol 400 | -- | 0.3 mg |
| Titanium Dioxide | -- | 0.56 mg |
| Purified Talc | -- | 1.14 mg |
| Total Weight | 420 mg | 922.25 mg |
| Assay Value--for diflunisal | 244 mg | 245.3 mg |
| --for tromethamine | -- | 552 mg |
| Dissolution--Q=80% in 30 minutes | Conform | -- |
| --in 30 minutes | -- | 101% |
| Weight Uniformity-- | Conform | -- |
| --Range | -- | 929-946 mg |
| --RSD | -- | 0.45% |

## Table 2

Summary of Pharmacokinetic Data[a]

| | Peak Plasma Level | | | | | Urinary Recovery | | |
|---|---|---|---|---|---|---|---|---|
| | $C_{max}$= mcg/ml | | Ratio | $T_{max}$= hrs | | mg | | Ratio |
| Subj. | D | DT | (DT/D) | D | DT | D | DT | (DT/D) |
| 1 | 32.23 | 35.68 | 1.11 | 2.0 | 2.0 | 124.21 | 114.27 | 0.92 |
| 2 | 27.00 | 39.37 | 1.46 | 1.5 | 1.0 | 150.58 | 127.07 | 0.84 |
| 3 | 25.99 | 33.57 | 1.29 | 1.5 | 1.5 | 161.73 | 160.69 | 0.99 |
| 4 | 35.72 | 32.21 | 0.90 | 3.0 | 1.5 | 114.31 | 105.72 | 0.92 |
| 5 | 31.92 | 22.42 | 0.70 | 1.5 | 0.75 | 170.79 | 140.35 | 0.82 |
| 6 | 26.53 | 49.00 | 1.85 | 4.0 | 1.0 | 148.02 | 146.49 | 0.98 |
| 7 | 31.70 | 31.68 | 1.00 | 3.0 | 2.0 | 170.92 | 127.75 | 0.74 |
| 8 | 27.64 | 44.08 | 1.59 | 4.0 | 2.0 | 91.52 | 167.20 | 1.82 |
| 9 | 31.96 | 37.97 | 1.19 | 1.5 | 1.0 | 191.34 | 154.96 | 0.81 |
| 10 | — | 36.55 | — | — | 0.5 | — | 113.66 | — |
| 11 | 41.59 | 37.72 | 0.91 | 3.0 | 2.0 | 132.97 | 116.90 | 0.87 |
| 12 | 32.91 | 25.37 | 0.77 | 1.5 | 2.0 | 165.93 | 156.13 | 0.94 |
| 13 | 29.86 | 45.74 | 1.53 | 3.0 | 0.75 | 118.49 | 125.17 | 1.05 |
| 14 | 30.60 | — | — | 2.0 | — | 142.13 | -- | — |
| 15 | 37.14 | 50.96 | 1.37 | 1.5 | 0.75 | 93.27 | 119.79 | 1.28 |
| 16 | 37.15 | 38.02 | 1.02 | 1.0 | 1.0 | 165.08 | 171.70 | 1.03 |
| 17 | 24.70 | 27.43 | 1.11 | 4.0 | 3.0 | 165.94 | 140.19 | 0.84 |
| 18 | 30.72 | 33.63 | 1.09 | 6.0 | 0.75 | 148.91 | 160.03 | 1.07 |
| Mean | 31.50 | 36.55 | 1.14* | 2.6 | 1.4 | -- | -- | 0.97** |
| S.D. | ±4.52 | ±7.85 | 0.98 | ±1.3 | ±0.7 | | | 0.87 |
| | | | 1.31 | | | | | 1.09 |

a D = DOLOBID Tablets (Treatment A); DT = diflunidal-tromethamine (treatment B)

* Geometric mean; values in parentheses indicate 95% C.I.

** Corrected for assayed dose.

In the presence of tromethamine, peak plasma levels of diflunisal following oral administration were not affected; however, peak time was improved. Urinary recovery of diflunisal remained unchanged. This test shows that the time to peak bioavailability of the diflunisal/tromethamine admixture is reduced from 2.6 hours (peak bioavailability of diflunisal alone) to 1.4 hours, so that an earlier clinical onset with the diflunisal/tromethamine admixture is anticipated.

EXAMPLE 2

This was an open-label, randomized four-way crossover study in 16 healthy male volunteers. Each subject received the following treatments, which were separated by at least 6 days.

Treatment A:

One DOLOBID 500 mg tablet

Treatment B:

One diflunisal/tromethamine 500/417 mg tablet

Treatment C:

One diflunisal/tromethamine 500/538 mg tablet

Treatment D:

One diflunisal/tromethamine 500/670 mg tablet

Plasma samples were collected for diflunisal assay at 0, 15, 30, 45, 60, 75, 90, 105, 120, 140, 160, 180, 210, and 240 minutes. Specimens were stored at -15°C until time of analysis.

Freshly voided urine was collected at zero hour (pre-dose), and at 0-1, 1-2, 2-3, and 3-4 hours post-dose. The pH of each specimen was immediately measured and recorded.

Table 1 lists the physicochemical properties of the four formulations tested in the study.

Mean plasma data, shown in Table 2, indicate a faster absorption of diflunisal in the presence of tromethamine, as manifested by a more rapid rise in the initial plasma concentrations.

Table 3 lists the individual peak plasma concentration ($C_{max}$) and time to the plasma peak ($T_{max}$). In general, there was no change in the $C_{max}$. However, there was a decrease in the $T_{max}$. The decrease averaged 0.67 ± 0.69, 0.56 ± 1.06, and 0.71 ± 0.74 hr following the administration of 500/417 mg, 500/538 mg, and 500/670 mg, respectively (Table 4).

The changes in diflunisal plasma concentrations as a result of faster diflunisal absorption are summarized in Table 5. For all three tromethamine-containing formulations, the higher initial plasma levels are accompanied by a more rapid decline of plasma levels beginning at approximately 2 hours post-treatment.

Table 1

Physicochemical Properties of the Test Formulations

| Treatment | B | C | D | A |
|---|---|---|---|---|
| Diflunisal | 500 mg | 500 mg | 500 mg | 500 mg |
| Tromethamine | 417 mg | 538 mg | 670 mg | — |
| Avicel PH 101 | 50 mg | 50 mg | 50 mg | 87 mg |
| Starch 1500 | 75 mg | 75 mg | 75 mg | 208 mg |
| Klucel LF | — | — | — | 17 mg |
| Avicel PH 101 | 40 mg | 40 mg | 40 mg | — |
| Purified Talc | 30 mg | 30 mg | 30 mg | — |
| Magnesium Stearate | 10 mg | 10 mg | 10 mg | 10 mg |
| Hydroxypropylmethyl Cellulose | 14.30 mg | 14.30 mg | 14.30 mg | 4.20 mg |
| Klucel LF | — | — | — | 4.20 mg |
| FD&C Yellow #6 Aluminium lake | — | — | — | 1.50 mg |
| Polyethylene Glycol | 0.85 mg | 0.85 mg | 0.85 mg | — |
| Titanium Dioxide | 1.60 mg | 1.60 mg | 1.60 mg | 0.55 mg |
| Purified Talc | 3.25 mg | 3.25 mg | 3.25 mg | 3.55 mg |
| Tablet Weight | 1142 mg | 1263 mg | 1395 mg | 834 mg |
| Assay Content: | | | | |
| Diflunisal | 507 mg | 508 mg | 511 mg | 494 mg |
| Tromethamine | 410 mg | 524 mg | 661 mg | — |
| Mean Tablet Weight | 1138 mg | 1263 mg | 1395 mg | 834 mg |
| % Dissolution, at: | | | | |
| 15 min. | 44 | 55 | 56 | — |
| 30 min. | 89 | 97 | 97 | 98 |
| 45 min. | 99 | 100 | 100 | — |
| Disintegration time, min. | <15 | <12 | <16 | <7 |

Table 2

Plasma Level of Diflunisal (Mean ± S.D.)

Following 500 mg of Diflunisal and

Varying Amounts of Tromethamine

| | Plasma Level, µg/ml | | | |
|---|---|---|---|---|
| Treatment Time, hr | A | B | C | D |
| 0.25 | 5.3 ± 8.9 | 9.0 ± 8.0 | 8.4 ± 4.3 | 11.1 ± 8.2 |
| 0.5 | 18.0 ± 15.3 | 29.3 ± 25.8 | 22.6 ± 10.3 | 31.1 ± 14.7 |
| 0.75 | 33.7 ± 21.5 | 46.4 ± 30.7 | 42.9 ± 18.0 | 45.4 ± 18.3 |
| 1 | 42.2 ± 19.4 | 53.7 ± 27.2 | 55.5 ± 20.0 | 59.1 ± 18.3 |
| 1.25 | 52.6 ± 16.3 | 61.7 ± 21.8 | 61.3 ± 16.1 | 63.9 ± 16.6 |
| 1.5 | 59.1 ± 15.6 | 63.2 ± 19.6 | 64.3 ± 20.7 | 65.0 ± 17.3 |
| 1.75 | 63.2 ± 19.5 | 63.8 ± 20.2 | 62.4 ± 15.5 | 69.4 ± 16.7 |
| 2 | 64.9 ± 19.9 | 63.3 ± 15.7 | 64.5 ± 14.4 | 63.4 ± 15.4 |
| 2.33 | 61.6 ± 17.9 | 62.2 ± 15.8 | 61.3 ± 13.4 | 61.0 ± 11.8 |
| 2.67 | 63.3 ± 24.4 | 60.4 ± 11.9 | 58.6 ± 13.3 | 60.3 ± 15.9 |
| 3 | 59.3 ± 14.9 | 56.2 ± 12.5 | 56.5 ± 16.9 | 53.1 ± 10.9 |
| 3.5 | 55.7 ± 12.5 | 53.9 ± 10.2 | 51.2 ± 13.7 | 52.1 ± 10.0 |
| 4 | 54.6 ± 11.6 | 50.6 ± 10.9 | 49.9 ± 11.5 | 46.8 ± 10.6 |

## Table 3

**Observed Maximum and Time of Maximum Concentration of Diflunisal in Plasma Following 500 mg Dosages of Diflunisal in a Tablet Formulation Containing Differing Amounts (mg) of Tromethamine**

| | $C_{max}$, µg/ml | | | | $T_{max}$, hr | | | |
|---|---|---|---|---|---|---|---|---|
| Subject | A | B | C | D | A | B | C | D |
| 1 | 92.8 | 109.9 | 103.1 | 99.5 | 2.0 | 1.50 | 1.50 | 1.75 |
| 2 | 84.5 | 80.5 | 66.5 | 78.6 | 2.33 | 2.00 | 1.75 | 1.50 |
| 3 | 73.7 | 76.3 | 70.6 | 95.8 | 4.00 | 3.00 | 1.50 | 1.75 |
| 4 | 74.1 | 85.9 | 77.5 | 80.2 | 2.00 | 1.00 | 1.50 | 1.00 |
| 5 | 106.0 | 104.2 | 98.2 | 104.9 | 1.75 | 1.00 | 1.50 | 1.25 |
| 6 | 70.4 | 51.6 | 56.1 | 70.0 | 3.00 | 1.50 | 2.33 | 1.50 |
| 7 | 81.5 | 85.1 | 76.7 | 67.8 | 1.25 | 1.25 | 1.00 | 1.25 |
| 8 | 72.9 | 101.1 | 70.5 | 76.1 | 2.00 | 0.75 | 2.00 | 1.75 |
| 9 | 132.4 | 90.8 | 82.0 | 103.9 | 2.66 | 2.33 | 2.00 | 2.66 |
| 10 | 74.5 | 69.8 | 66.9 | 74.4 | 1.00 | 1.75 | 2.00 | 0.75 |
| 11 | 83.1 | 73.6 | 91.7 | 85.1 | 2.33 | 2.66 | 1.00 | 1.50 |
| 12 | 67.3 | 67.9 | 74.6 | 71.8 | 1.50 | 1.25 | 1.00 | 1.00 |
| 13 | 46.9 | 78.9 | 32.3 | 48.7 | 2.66 | 0.75 | 4.00 | 2.66 |
| 14 | 65.7 | 68.7 | 82.3 | 62.8 | 2.66 | 2.00 | 1.50 | 2.66 |
| 15 | 33.9 | 60.7 | 62.3 | 66.9 | 4.00 | 2.66 | 1.25 | 1.75 |
| 16 | 79.3 | 104.8 | 74.1 | 67.4 | 2.66 | 1.75 | 3.00 | 1.75 |
| Mean | 77.4 | 81.9 | 74.1 | 78.4 | 2.36 | 1.70 | 1.80 | 1.66 |
| S.D.[a] | 22.1 | 16.9 | 16.8 | 15.9 | 0.85 | 0.70 | 0.79 | 0.59 |

[a] Standard deviation

**Table 4**

**Change in $T_{max}$ from Diflunisal Tablet Treatment to Diflunisal/Tromethamine Tablet Treatments**

| | $\Delta T^a_{max}$, hr | | |
|---|---|---|---|
| Subject No. | B | C | D |
| 1 | 0.50 | 0.50 | 0.25 |
| 2 | 0.33 | 0.58 | 0.83 |
| 3 | 1.00 | 2.50 | 2.25 |
| 4 | 1.00 | 0.50 | 1.00 |
| 5 | 0.75 | 0.25 | 0.50 |
| 6 | 1.50 | 0.67 | 1.50 |
| 7 | 0 | 0.25 | 0 |
| 8 | 1.25 | 0 | 0.25 |
| 9 | 0.33 | 0.66 | 0 |
| 10 | -0.75 | -1.00 | 0.25 |
| 11 | -0.33 | 1.33 | 0.83 |
| 12 | 0.25 | 0.50 | 0.50 |
| 13 | 1.91 | -1.34 | 0 |
| 14 | 0.66 | 1.16 | 0 |
| 15 | 1.34 | 2.75 | 2.25 |
| 16 | 0.91 | -0.34 | 0.91 |
| Mean | 0.67 | 0.56 | 0.71 |
| S.D. | 0.69 | 1.06 | 0.74 |

[a] $\Delta T_{max} = T_{max}$ (diflunisal treatment) - $T_{max}$ (diflunisal/tromethamine treatment)

Table 5

Increase in Diflunisal Plasma Level (Mean ± S.D.)

Following 500 mg Diflunisal and Varying Amounts of Tromethamine

| Time, hrs. | $\Delta Cp^a$, µg/ml B | C | D |
|---|---|---|---|
| 0.25 | 3.7 ± 8.3 | 3.1 ± 8.0 | 5.8 ± 7.9 |
| 0.5 | 11.3 ± 24.6 | 4.6 ± 15.9 | 13.1 ± 19.4 |
| 0.75 | 12.7 ± 32.0 | 9.2 ± 21.2 | 11.7 ± 23.9 |
| 1 | 11.5 ± 34.7 | 13.3 ± 25.5 | 16.8 ± 23.1 |
| 1.25 | 9.1 ± 25.5 | 8.7 ± 18.2 | 11.3 ± 18.2 |
| 1.5 | 4.1 ± 20.9 | 5.2 ± 18.9 | 5.9 ± 18.6 |
| 1.75 | 0.6 ± 21.8 | -0.9 ± 19.1 | 6.2 ± 22.6 |
| 2 | -1.6 ± 12.3 | -0.4 ± 12.8 | -1.5 ± 19.2 |
| 2.33 | 0.7 ± 14.8 | -0.3 ± 14.9 | -0.6 ± 14.1 |
| 2.67 | -2.9 ± 18.9 | -4.7 ± 18.4 | -3.0 ± 16.2 |
| 3 | -3.1 ± 18.0 | -2.8 ± 18.4 | -6.2 ± 14.7 |
| 3.5 | -1.8 ± 9.0 | -4.5 ± 13.5 | -3.7 ± 8.9 |
| 4 | -4.0 ± 12.2 | -4.6 ± 10.9 | -7.7 ± 9.0 |

[a] ΔCp = Cp (diflunisal/tromethamine treatment) - Cp (diflunisal treatment)

Summary of the Results

Compared to the DOLOBID treatment, all three diflunisal/tromethamine formulations (500/417 mg, 500/538 mg, 500/670 mg) showed faster absorption of diflunisal as indicated by shorter times to the peak plasma concentration. The degree of improvement was similar among the three formulations. Although tromethamine did not enhance diflunisal peak plasma concentration, it produced higher initial plasma levels prior to reaching the plasma peak. The greatest increase was produced by the 500/670 mg treatment. During the initial 0-2 hour time interval, the 500/670 mg treatment also produced a slightly greater number of subjects with higher drug concentrations.

EXAMPLE 3

Following substantially the same procedure as described in Examples 1 and 2, an open-label, randomized four-way crossover study in 16 healthy male volunteers is carried out. Each subject received following treatments which are separated by at least 6 days.

Treatment A:

One DOLOBID 500 mg tablet (formulation as set out under A in Table 1 of Example 2)

Treatment B:

One diflunisal/tromethamine 500/400 tablet

Treatment C:

One diflunisal/tromethamine 500/300 tablet

Treatment D:

One diflunisal/tromethamine 500/200 tablet

Treatment E:

One diflunisal/tromethamine 500/100 tablet
The composition of the tablets are listed in Table 1 below.

**Table 1**

**Tablet Compositions for**

**Treatments B, C, D and E**

| Ingredients | Treatment B | Treatment C | Treatment D | Treatment E |
|---|---|---|---|---|
| Diflunisal | 500 mg | 500 mg | 500 mg | 500 mg |
| Tromethamine | 400 mg | 300 mg | 200 mg | 100 mg |
| Microcrystalline Cellulose (Avicel PH101) | 90 mg | 90 mg | 90 mg | 90 mg |
| Pregelatinized Starch (Starch 1500) | 50 mg | 50 mg | 50 mg | 50 mg |
| Cross Linked Polyvinylpyrrolidone (Plasdone XL) | 40 mg | 40 mg | 40 mg | 40 mg |
| Talc | 10 mg | 10 mg | 10 mg | 10 mg |
| Magnesium Stearate | 10 mg | 10 mg | 10 mg | 10 mg |
| Hydroxypropylmethylcellulose (Methocel) | 10.73 mg | 10.73 mg | 10.73 mg | 10.73 mg |
| Polyethylene Glycol | 0.637 mg | 0.637 mg | 0.637 mg | 0.637 mg |
| Titanium Dioxide | 1.20 mg | 1.20 mg | 1.20 mg | 1.20 mg |
| Talc | 2.438 mg | 2.438 mg | 2.438 mg | 2.438 mg |
| Coated Tablet Weight | 1.115 g | 1.105 g | 915 mg | 815 mg |

Upon conclusion of the treatment, results similar to Example 2 is expected for treatment B and C but to a lesser extent for treatments D and E.

## Claims

1. An admixture comprising from 125 to 500 mg of diflunisal and from 50 to 424 mg of tromethamine.
2. The admixture of Claim 1, comprising 200 to 500 mg of diflunisal and 100 to 424 mg of tromethamine.
3. The admixture of Claim 1 wherein the weight ratio of diflunisal to tromethamine is 5:1 to 5:4.
4. The admixture of Claim 1 comprising:
   (a) 500 mg of diflunisal and 400 mg of tromethamine;
   (b) 500 mg of diflunisal and 300 mg of tromethamine;
   (c) 500 mg of diflunisal and 200 mg of tromethamine; or
   (d) 500 mg of diflunisal and 100 mg of tromethamine.
5. A pharmaceutical composition for treating pain and inflammation comprising the admixture of Claim 1 and a pharmaceutically acceptable carrier thereof.
6. The composition of Claim 5 comprising 200 to 500 mg of diflunisal; 100 to 424 mg of tromethamine; and a pharmaceutically acceptable carrier.
7. The composition of Claim 5 comprising an admixture of diflunisal and tromethamine and a pharmaceutically acceptable carrier wherein the weight ratio of diflunisal and tromethamine is 5:1 to 5:4.
8. The composition of Claim 5 comprising an admixture of (a) 500 mg of diflunisal and 400 mg of tromethamine; (b) 500 mg of diflunisal and 300 mg of tromethamine; (c) 500 mg of diflunisal and 200 mg of tromethamine; or (d) 500 mg of diflunisal and 100 mg of tromethamine; and a pharmaceutically acceptable carrier.
9. The use of the admixture of Claim 1 for the preparation of a medicament useful for the treatment of inflammation and pain.
10. The use of Claim 9 wherein an admixture of 200 to 500 mg of diflunisal and 100 to 424 mg of tromethamine is administered.
11. The use of Claim 9 wherein the admixture comprises diflunisal and tromethamine in the weight ratio of 5:1 to 5:4.
12. The method of Claim 9 wherein the admixture comprises (a) 500 mg of diflunisal and 400 mg of tromethamine; (b) 500 mg of diflunisal and 300 mg of tromethamine; (c) of diflunisal and 200 mg of tromethamine; or (d) mg of diflunisal and 100 mg of tromethamine.

Claims for the following contracting States: AT, GR, ES.

1. A process for preparing an admixture comprising mixing from 125 to 500 mg of diflunisal and from 50 to 424 mg of tromethamine.
2. The process of Claim 1 comprising mixing 200 to 500 mg of diflunisal and 100 to 424 mg of tromethamine.
3. The process of Claim 1, wherein the weight ratio of diflunisal to tromethamine is 5:1 to 5:4.
4. The process of Claim 1 comprising mixing:
   (a) 500 mg of diflunisal and 400 mg of tromethamine;
   (b) 500 mg of diflunisal and 300 mg of tromethamine;
   (c) 500 mg of diflunisal and 200 mg of tromethamine; or
   (d) 500 mg of diflunisal and 100 mg of tromethamine.
5. A process for preparing a pharmaceutical composition for treating pain and inflammation comprising mixing the admixture of Claim 1 and a pharmaceutically acceptable carrier thereof.
6. The process of Claim 5 comprising mixing 200 to 500 mg of diflunisal; 100 to 424 mg of tromethamine; and a pharmaceutically acceptable carrier.
7. The process of Claim 5 comprising mixing an admixture of diflunisal and tromethamine and a pharmaceutically acceptable carrier wherein the weight ratio of diflunisal and tromethamine is 5:1 to 5:4.
8. The process of Claim 5 comprising mixing (a) 500 mg of diflunisal and 400 mg of tromethamine; (b) 500 mg of diflunisal and 300 mg of tromethamine; (c) 500 mg of diflunisal and 200 mg of tromethamine; or (d) 500 mg of diflunisal and 100 mg of tromethamine; and a pharmaceutically acceptable carrier.

European Patent Office

# EUROPEAN SEARCH REPORT

Application Number

EP 87 30 5528

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 017 102 (MERCK)<br>* Claim 1 *<br>--- | 1 | A 61 K 31/615<br>A 61 K 31/19 //<br>(A 61 K 31/19<br>A 61 K 31:13 ) |
| E | EP-A-0 230 125 (MERCK)<br>* Claims 1-6 *<br>----- | 1-12 | |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 13-09-1988 | PEETERS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document